# EUROPEAN PATENT APPLICATION

(11) **EP 1 393 743 A1**
(43) Date of publication of application: **03.03.2004**
(21) Application number: 03024328.1
(22) Date of filing: 22.01.2002
(51) Int. Cl.: A61K 38/49, A61K 38/08, A61K 38/10, A61K 39/395, G01N 33/53, G01N 33/569, G01N 33/574, A61P 31/18

(54) **Agents modulating UPA/UPAR activity for the treatment of AIDS**

(30) Priority: 25.01.2001 US 263198 P
(62) Divisional of application: 02710810.9
(71) Applicant: FONDAZIONE CENTRO SAN RAFFAELE DEL MONTE TABOR, 20132 Milano (IT)
(72) Inventor: Blasi, Francesco, 20132 Milano (IT); Sidenius, Nicolai, 20132 Milano (IT); Poli, Guido, 20132 Milano (IT); Alfano, Massimo, 20132 Milano (IT)
(74) Representative: Banfi, Paolo

(57) **Abstract**

The invention relates to the use of pro-uPA, derivatives and fragments thereof, or of activated uPAR molecules, for the diagnosis and therapy of HIV-infection.

## Description

The present invention relates to methods and agents modulating uPA/uPAR function, useful for the treatment of uPA/uPAR-related diseases. More precisely, the invention provides the use of pro-uPA, derivatives and fragments thereof, or of activated uPAR molecules, for the diagnosis and therapy of HIV-infection.

### Background of the invention

### Cell migration and disease

The human body responds to pathogenic stimuli by locally producing specific inflammatory molecules and recruiting specialized cells from blood and nearby tissues. This occurs in almost all diseases, and contributes in most cases to the general defenses of the organism. However, sometimes the inflammatory reaction, or the lack thereof, also contribute to the disease itself. Neutrophils, monocyte-macrophages, lymphocytes, endothelial cells, fibroblasts are attracted by specific migratory stimuli and build up a defense response that causes the destruction and elimination of the disease agent or organism. A series of severe pathological entities can be ascribed to excess or deficiency of the migratory cell response. For example, immunodeficiencies may be caused by a failure of the inflammatory cells to rush to the damaged or infected site. Cell recruitment in itself, on the other hand, may cause destructive pathologies in which autologous cells attack and destroy cells and tissues, as in some auto-immune diseases. It would be particularly advantageous to be able to know in advance such situations in order to take special precautions.

A special example among diseases is cancer. In this disease, a deficiency of the host lymphatic cells may be at the basis of the lack of humoral or cellular response against the cancer cells themselves, as it would be expected since cancer cells express very peculiar antigens. On the other hand, cancer cells have the particular ability to invade nearby tissues and to metastasize at a distance; this process, however, is due not only to a property of cancer cells. Indeed, non-malignant stromal host cells do actively participate in establishing the malignant and hence the destructive and invasive phenotype. While the mechanisms involved are still largely unknown, it is clear that a cooperation of stromal and cancer cells is required for the malignant phenotype, and that the stromal cells both respond to stimuli arriving from the cancer cells and signal to cancer cells. The pharmacological block of the invasive phenotype of cancer, must therefore rely on actions on both cancer and stromal cells. Again, it would be particularly advantageous to be able to know in advance the specific contributions of each type of cells.

Another special example is AIDS, a disease caused by HIV-1 and characterized by a progressive and severe immune-deficiency and by a series of resultant deadly infections, tumors etc., caused by the immuno-deficiency itself.

Finally, infectious diseases have to be considered, since foreign bacteria recruit neutrophils and monocytes/macrophages to the site of infection.

### Plasminogen activator of the urokinase-type (uPA) and its receptor (uPAR)

Urokinase-type plasminogen activator (uPA) is a serine protease that activates plasminogen to plasmin, a broad-spectrum serine protease itself. Plasmin is an important protease in fibrinolysis and its activity appears to be required also in wound healing.

UPA is synthesized as an inactive precursor (pro-uPA) that undergoes proteolytic activation to uPA. Pro-uPA (and uPA) bind with high affinity (Kd of 0.1 nM) (Stoppelli, 1985; Vassalli, 1985) to a specific plasma membrane receptor (uPAR, CD87) that localizes its activity to the cell surface, thus restricting the area of activation of plasminogen (Stephens et al., 1989). The molecule of uPA can be divided in two fragments, each endowed with a specific activity: the amino terminal fragment (ATF) has the receptor-binding activity, the carboxy-terminal fragment (also called low molecular weight urokinase) has the full proteolytic activity.

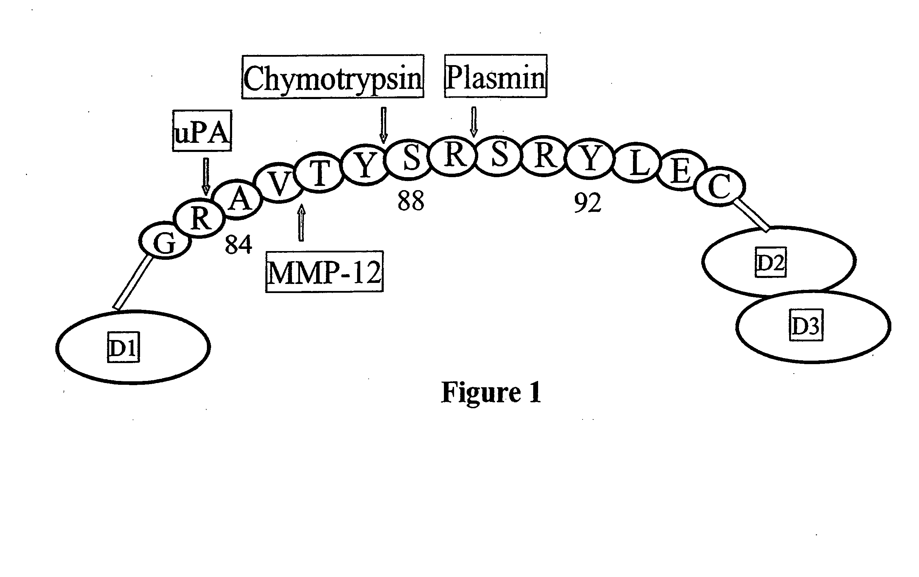

UPAR (Dano, 1990) is a molecule present on the cell surface and formed by three repeats, characterized by a specific disulfides-pattern (see Figure 1 for a scheme). The three repeats, of about 90 residues each, are connected by two linker regions and define specific protein domains. The amino-terminal domain, D1, has been shown to bind uPA directly, although other areas of the receptor (like domain D3) are also relevant in contacting uPA. uPAR is bound to the cell surface with a specific glycosyl-phosphatidylinositol (GPI) anchor, and hence lacks a trans-membrane and an intracellular moiety which would be typical of other receptors. Importantly (see below), the linker region between domain D1 and D2 contains a sequence, AVTYSRSRYLEC, to which a chemotactic epitope has been mapped (Blasi, 1997; Fazioli, 1997; Nguyen, 2000).

### Ligands of uPAR

In addition to specifically binding uPA, uPAR is able to also bind vitronectin, hence being able to provide an anchorage to the cells when plated on vitronectin (Deng et al., 1996; Hoyer-Hansen, 1997; Sidenius, 2000; Waltz, 1994). In addition, uPAR has been reported to interact with other molecules, like thrombospondin (Higazi et al., 1996), kininogen (Colman et al., 1997), although at lower affinity. Important among these appear to be the interactions with integrins (Simon, 2000; Tarui, 2000; Waltz et al., 1997; Wei et al., 1996; Xue et al., 1994; Xue et al., 1997; Yebra et al., 1996). The interaction with integrins appears to have signaling properties, since it can affect cell adhesion (Wei et al., 1996) and cell migration (Degryse, 1999; Degryse, 2001; Yebra et al., 1996). Finally, interactions with other cell surface proteins like the mannose-6-phosphate receptor (Nykjaer, 1998) and the uPARAP (Behrendt, 2000) have been reported.

### UPAR and signaling

In the last few years it has been convincingly shown that uPAR is a signaling receptor when it is activated by uPA. In this respect, the catalytic activity of uPA in some cases does not appear to be required. Binding of uPA to uPAR induces cell migration, cell adhesion and proliferation (Blasi, 1997; Chapman, 1997; Ossowski, 2000). A. Cell migration appears to be induced at physiological expression levels of uPAR, while cell adhesion and proliferation appear to require high levels of receptors, as those present in cancer or in transfected cells (Ossowski, 2000). In line with this possibility, mice lacking uPA or uPAR have a strong immunodeficient phenotype due to lack of migration of several inflammatory cells, and are unable to fight infection by *C. neoformans* and *P. aeruginosa* (Gyetko, 1996; Gyetko, 2000). The migration-promoting activity of uPAR requires binding of uPA and is mediated by a pertussis toxin-sensitive receptor and requires the activation of a Src-family tyrosine kinase, and of ERKs (Fazioli, 1997; Nguyen, 2000; Ossowski, 2000; Resnati, 1996). Importantly, infection with **B.** The cell adhesion properties of uPAR are mediated by a lateral interaction with at least some of the cytoskeleton-engaged integrins and are mediated by caveolin (Wei et al., 1996; Wei et al., 1999). C. The proliferation properties, on the other hand, depend on a constitutive engagement of an α4-integrin by uPAR, resulting in constitutive activation of the proliferation-promoting ERK and inhibition of the growth-inhibiting p38-MAP kinase (Aguirre Ghiso et al., 1999; Ossowski, 2000).

### UPAR and cell migration

Exogenous uPA induces chemotaxis in a variety of cells (Boyle, 1987; Degryse, 1999; Fibbi, 1988; Gudewicz, 1987; Gyetko, 1994; Nguyen, 2000; Resnati, 1996; Webb et al., 2000). In-depth studies have shown that uPA binding transforms uPAR into a ligand for a cell surface protein which can transduce its signal (Blasi, 1997). Upon binding of u-PA, a peptide epitope of u-PAR, which is otherwise hidden in the context of the molecule, comes at the surface of the molecule or becomes available because of cleavage of uPAR. The peptide has the sequence AVTYSRSRYLEC (Figure 1), and is located in the region linking domain D1 to D2 and endowed with chemotactic activity (Blasi, 1997; Fazioli, 1997). Recombinant fragments of u-PAR containing at least the SRSRY sequence ("activated" uPAR) induce migration in uPAR-lacking cells (Blasi, 1997; Fazioli, 1997). The chemotactic epitope of u-PAR remains active whether placed at the N- or at the C-terminal end of a recombinant fragment. Indeed, both domain D1-SRSRY or SRSRY-domain D2-D3 are both chemotactically active (Blasi, 1997; Fazioli, 1997). Synthetic peptides containing at least the SRSRY sequence can efficiently substitute for uPA-uPAR interactions or for the addition of exogenous activated uPAR fragments (B. Degryse, 1999; Blasi, 1997; Fazioli, 1997).

### The uPAR-adapter

Since uPAR lacks an intracellular domain, a trans-membrane adapter has been suggested to mediate the signaling effects of uPA/uPAR (Resnati, 1996). The best evidence for the existence of an adapter is the finding that in cells lacking uPAR, a soluble modified form of uPAR can directly act as a chemoattractant. A sequence between domain D1 and D2 of uPAR has been shown to be responsible for this activity. In fact, a synthetic peptide corresponding to this region or the uPAR fragment carrying this peptide at the amino terminus (henceforth called "D2D3₈₈₋₂₇₈") has a very potent chemotactic activity and reproduces the signaling events evoked by uPA (Blasi, 1997; Fazioli, 1997; Nguyen, 2000). Chemotaxis induced by uPA and D2D3₈₈₋₂₇₉ in peripheral blood monocytes, in THP-1 myeloid precursors leukemic cells, cancer cells and smooth muscle cells is pertussis-toxin sensitive and induces Hck and MEK phosphorylation. The latter are required for activity (Degryse, 2001; Nguyen, 2000; Resnati, 1996; Webb et al., 2000).

### U-PAR fragments and human diseases

In addition to an intact su-PAR (Sier, 1998), fragments of su-PAR are often found in cancer tissue and in cell lines. From their size and from their antibody reactivity these fragments correspond roughly to either a released domain D1 or to a released or cell-bound domain D2-plus-D3 (Hoyer-Hansen, 1997; Hoyer-Hansen, 1992; Mustjoki, 2000; Solberg, 1994). Moreover, these fragments are also found in the conditioned media of, for example, U937 cells and their abundance relative to the intact su-PAR appears to be controlled (Sidenius, 2000).

The discovery that those fragments of u-PAR that contain at their N- or C-termini the SRSRY minimal epitope have potent chemotactic activity (Blasi, 1997; Fazioli, 1997), demonstrates that some of the fragments which are produced in vivo may in fact be "activated" u-PAR molecules. Their production in a given tissue can profoundly change the migratory potential of the cells present in that tissue. Hence, it is very important to identify if such in vivo u-PAR fragments represent in fact "activated" u-PAR. Such fragments have been found in serum or in tissues at low levels in the urine of normal individual (Sidenius, 2000) and at high levels in diseases such as leukemia (blood) (Mustjoki, 2000) and cancer (urine and tissues but not blood) (Sidenius, 2001), and may possibly be found in many others. In healthy individuals such fragments have never been found in the blood, and are present in very low amounts in their tissues and urine. However, the presence of the chemotactic epitope in these fragments cannot be evaluated except by complex methods involving protein purification and sequencing.

U-PAR is very resistant to proteases with the exception of the linker region between domain D1 and D2 (Behrendt, 1991). This region can be cleaved in vitro by several proteases. Cleavage in this region results in the production of two unequal fragments of u-PAR, one containing domain D1, the other domain D2-plus-D3. The nature of the cleaving protease will determine whether and how much of the chemotactic epitope is preserved in the u-PAR fragments. (Figure 1). For example, chymotrypsin cleaves between residue 87 and 88, leaving a D2-plus-D3 fragment with the SRSRYLEC amino terminus. This "activated" uPAR, when prepared in soluble form, has a very potent chemotactic activity on several cells and cell lines (Blasi, 1997; Fazioli, 1997). Also uPA itself cleaves uPAR, at residue 84, producing a D2-plus-D3 fragment with an amino terminal AVTYSRSRYLEC sequence (Hoyer-Hansen, 1997; Hoyer-Hansen, 1992). Again, this fragment is endowed with a very strong chemotactic activity. The synthetic peptide AVTYSRSRYLEC also has an extremely potent chemoattractant activity, such as shorter peptides that still contain the SRSRY sequence (Blasi, 1997; Fazioli, 1997). Alternatively, enzymes like plasmin can probably destroy the epitope as they cleave at both residue 90 and 92, i.e. within the SRSRY region (Resnati et al., 1996).

The fact that u-PAR fragments are produced in vivo, and that their level is increased in several diseases suggests that they can be part of the disease itself. Hence, it will be important to measure their level and their molecular nature.

### Release of soluble u-PAR in the circulation in human diseases

Many receptors are found in blood. U-PAR makes no exception. It has been first found in the blood and ascitic fluid of ovarian cancer patients (Pedersen, 1993) and subsequently in tissues, blood or even urine of many other types of cancer, including leukemias. In most cases high levels of released su-PAR can be related to poor prognosis (Stephens, 1999). Also in other diseases, su-PAR level is increased in tissues or blood: i.e. Rheumatoid Arthritis (Slot, 2000; Slot, 1999) and particularly in AIDS (see below). Release of soluble uPAR was also observed upon infection of cells to *Borrelia burgdorferi, Salmonella typhimurium* and *Streptococcus pyogenes* (Coleman JL, 2001).

### UPA/uPAR and AIDS

Multiple evidence ties together uPA and uPAR with the HIV and AIDS. First, uPA and uPAR ecpression on T lymphocytes and monocytes is modulated by HIV infection (Speth, 1998) and uPAR is an activation antigen in T cells and is increased in AIDS patients (Nykjær, 1994). Finally, it has been shown that uPA can directly cleave the viral gp 120 in the V loop, important for viral infection (Handley, 1996).

We have recently measured the level of soluble uPAR (suPAR) in the blood of a well defined, large cohort of HIV-positive individuals drawn prior to the introduction of the highly active anti-retroviral therapy regimens. In this cohort, the level of suPAR was found to be proportional to the severity of the disease state (i.e. serum-positive v. immuno-deficient v. AIDS) and was an extremely significant negative prognostic indicator (Sidenius, 2000). The prognostic significance of the levels of suPAR was independent of, and as indicative as, the decrease of CD4⁺ T-lymphocytes or a very high viremia. A low level of suPAR was found to be a good prognostic marker even in patients with high viremia and low CD4⁺ cells. Conversely, a high level of suPAR was a negative indicator even in patients with relatively high CD4⁺ cells and low viremia. The statistical significance of these correlations (Sidenius, 2000) suggests that the uPA/uPAR system may somehow intervene in the pathogenesis of AIDS.

### Description of the invention

### 1. Pro-uPA and its analogs/derivatives inhibit HIV-infection and the reactivation of latent HIV

The present invention relates to the effect of pro-uPA on the reactivation of latent HIV-1 or on the infection and replication of HIV-1 in human cells. U1 cells harbour a latent HIV-1 which can be reactivated by various cytokines. In these cells, the proenzyme form (pro-uPA) of urokinase plasminogen activator (uPA) prevents the reactivation of HIV replication induced by PMA or TNF-alpha, and synergizes with anti-TNF-alpha antibodies and with TGF-beta in totally blocking this reactivation. In addition, pro-uPA also blocks the entry/infection of HIV in peripheral blood mononuclear cells (PBMC) without affecting cell proliferation. In accordance with these results, we have found that the U937 cells clones which sustain a rapid and efficient proliferation of HIV upon infection ("plus" clones) display high levels of uPAR. On the contrary, other clones which show a much delayed and inefficient production of virus ("minus" clones) display a very low level of uPAR. Pro-uPA can be used together with other drugs to block HIV infection or its spreading.

### 2. Antibodies and ELISA to specifically recognize cleaved, "activated" forms of uPAR and to inhibit uPA/uPAR induced migration

Polyclonal antibodies raised against the chemotactically active sequence AVTYSRSRYLEC specifically recognize cleaved ("activated") forms of u-PAR containing residues 84 and/or 85, and/or 86, and/or 87 at the amino terminus. Antibodies generated by injecting the peptide AVTYSRSRYLEC into rabbits were found to be unable to recognize intact, full-length su-PAR. However, they identified cleaved form of D2-plus-D3 that contain at their N-termini the AVTYSRSRYLEC, or VTYSRSRYLEC or TYSRSRYLEC orYSRSRYLEC at the amino terminus. The antiserum did not recognize fragments with amino-terminal SRSRYLEC obtained by cleavage with chymotrypsin. An ELISA assay capable of specifically quantitating the concentration of D2-plus-D3 is also reported. Therefore, other antibodies (such as monoclonal antibodies) can be generated to specifically identify all possible fragments of D2 plus D3 relevant for human diseases.

The present invention provides a method to identify and quantitate the presence of activated forms of u-PAR, the receptor for u-PA, the urokinase-type plasminogen activator. The method comprises the production of antibodies that specifically identify the presence in tissues, in extracts, in biological fluids of natural or recombinant forms of u-PAR which contain at their termini the chemotactic peptide of u-PAR. The method also comprises the set-up of an ELISA (Enzyme-Linked Immuno-Sorbent Assay) to quantitatively assess in absolute or percentile terms the presence of said forms of u-PAR. The use of these method and reagents is important in a variety of diseases caused by deficient or excessive cell migration, where the production of activated forms of u-PAR is required.

### DESCRIPTION OF THE FIGURES

**Figure 1. -** A scheme of the structure of uPAR. This is composed of three domains, D1, D2 and D3. The linker region between domain D1 and D2 starts at Gly 83. UPA can cleave between residues Arg84 and Ala85, MMP-12 between Thr86 and Tyr87, chymotrypsin between Tyr87 and Ser88 and plasmin between Arg89 and Ser90. Domain D3 is connected to the cell surface by a GPI anchor.
**Figure 2. -** Pro-uPA and ATF, but not LMW uPA, inhibit HIV-1 reactivation induced by PMA (10 nM) in U1 cells. SuPAR blocks the effect of pro-uPA.Pro-uPA and other effectors added at zero time.
**Figure 3. -** The anti-HIV effect of proUPA is reverted by R3, R4 and R5 antibodies in U1 cells. Data are shown as average and STD of duplicates collected at peak of HIV expression (day 4), measured as RT activity (cpm/µl). a-TNP is an isotype-matched control.
**Figure 4. -** Pro-uPA inhibits TNF-alpha (1 ng/ml) induced reactivation of HIV-1 in U1 cells (A). Pro-uPA and anti-TNF-alpha antibodies (1 µg/ml) (B), or pro-uPA (10 nM) and TGF-beta (5 ng/ml) (C) synergize in inhibiting HIV-1 reactivation induced by PMA in U1 cells. Pro-uPA and all other effectors were added at zero time.
**Figure 5. -** uPA does not inhibit IL-6 induced reactivation of HIV-1 in U1 cells. IL-6 was used at 10 ng/ml.
**Figure 6. -** Pro-uPA has different effects on HIV-1 replication of human ... (A), ... (B) or ... (C). The reverse transcriptase activity (RT, cpm/µl)) was measured every 3-4 days for a total of 20-25 days.
Figure 7. - Pro-uPA has no effect on cellular thymidine incorporation (expressed in % of control, i.e. cells not receiving pro-uPA). The data are separated for ... (A), ... (B) or ... (C).
**Figure 8A. -** The ability of HIV-1 to replicate in different clones of U937 cell correlates with the level of expression of uPAR, as measured by ELISA.The "plus" clones (+) are those that replicate very efficiently and rapidly (2-3 weeks). The "minus" (-) clones replicate very inefficiently and slowly (6 to 7 weeks). PMA, when used, was at 10 nM.
**Figure 8B. -** Immuno-blot analysis of extracts of untreated or PMA-treated (10 nM) U937cells clones. cSB and c10 are "plus" clones (high replication of HIV-1. c12 and c34 are "minus" clones (low replication efficiency). at two
**Figure 9. -** Use of the anti-peptide 3 antibody to identify specifically activated uPAR fragments. Full-length human suPAR and D2D3 fragments with different amino termini were run in SDS-PAGE, blotted and tested with two different antisera. D2D3(88) is the fragment generated by cleavage of suPAR with chymotrypsin. D2D3(84)FLAG and D2D3(92)FLAG have been generated by recombinant DNA technology and also contain a carboxy-terminal FLAG epitope.
**Figure 10. -** Enzyme-linked ImmunoSorbent Assay (ELISA) of suPAR and D2D3 fragments with different amino termini. SuPARcutCT is a D2D3 preparation obtained by cleavage of suPAR with chymotrypsin and purification (i.e. amino terminus at residue 89). suPARcutMMP12 isa D2D3 fragment obtained by cleavage with MMP-12 and purification.

### EXAMPLE 1

### Pro-urokinase (pro-uPA) and its derivatives uPA and ATF, inhibit the replication of HIV-1 in latently infected monocytoid cell lines and in freshly infected human blood cells.

Latent HIV infected cells provide a cellular population resistant to anti-retroviral therapy and represent the mechanism for lifelong persistence of HIV (Finzi et al., 1999). The promonocytic U937 cell line is one of the most utilized models for studies on in vitro HIV infection. It displays both CD4 and CXCR4 and, therefore, can be infected by X4 viruses. From this cell line has been derived the chronically infected cell line named "U1", containing two copies of integrated proviruses, and broadly used as a model of viral latency and reactivation (Folks et al., 1988). The U1 promonocytic cell line is one of the most thoroughly characterized models of post-integration latency. It was obtained from a population of U937 cells surviving the cytopathic effect of acute infection by HIV-1_{LAI/IIIB} (X4), and contains two copies of integrated proviruses (Folks et al., 1987; Folks et al., 1988). In the absence of stimulation, U1 cells expresses low levels of multiply spliced 2 kb HIV-1 transcript (Butera et al., 1994) encoding the regulatory proteins Tat, Rev and Nef (Pomerantz et al., 1990). Several studies have subsequently demonstrated that the state of relative latency in U1 cells is a consequence of defective Tat function (Cannon et al., 1994; Emiliani et al., 1996). High levels of virus production can be rapidly induced by stimulation of U1 cells with pro-inflammatory cytokines, such as tumor necrosis factor-α (TNF-α), IL-6, IL-1β (Vicenzi et al., 1997), through the activation of MAPK and consequent stimulation of AP-1 and NF*-k*B (Yang et al., 1999). Alternatively, reactivation of virus replication can also be achieved by production of endothelium-derived soluble factors including MCP-1, TNF-α and IL-6 (Borghi et al., 2000), and by chemical agents such as phorbol-myristate acetate (PMA) (Folks et al., 1988). In addition to HIV-inducing agents, inhibitor agents have also been reported. These include the interferon-inducible double-stranded RNA dependent p68 protein kinase that inhibits TNF-α-induced HIV replication (Muto et al., 1999), and TGF-β that inhibits PMA-induced HIV expression (Poli et al., 1991).

It has been previously reported that different clones of U937 cells, originated by limiting dilution, could be divided in two distinct categories named "Plus" and "Minus", to describe their differential ability of sustaining productive HIV infection. Infection of plus clones typically shows a peak of virus replication between the second and the third weak, whereas minus clones are substantially delayed (typically, the peak of virus replication is observed after 6-9 weeks from infection) and sometimes with very low levels of virus production (Franzoso et al., 1994). We have measured the levels of uPAR in "plus" and "minus" clones and correlated thse values with the ability to sustain viral replication. We find that low levels of uPAR correlate with high levels of viral replication.

### Materials and Methods

### Reagents

uPA, uPAR, suPAR, LMW-uPA, ATF, R2, R3, R4, R5 antibodies have been thoroughly described in the literature (Blasi, 1994; Rønne, 1991). Phorbol-myristate acetate (PMA) (Sigma, Milano, Italy) was used at 10⁻⁸ M, recombinant IL-6, TGF-β, TNF-α and polyclonal antibody antiTNF-α and isotype were purchased from R&D Systems (Minneapolis, MN) and used at the final concentrations of 10, 5 and 1 ng/ml, and 1µg/ml respectively, as previously reported (Poli, 1990; Poli et al., 1990).

### Cell proliferation

Cell proliferation was assessed using ³H-thymidine uptake assay. One µCi of ³H-thymidine was added to 2×10⁴ cells in 100 µl medium and incubated 16 h at 37°C, 5% CO₂. Cells were harvested and counted in a β-counter (TopCount, Packard, Downers Grove, IL).

### Cell lines

U1 cells were stimulated at the density of 2×10⁵ cells per ml in RPMI 1640 (Whittaker M.A. Bioproducts, Walkerville, Md.) containing 10% FCS. Cells were preincubated with uPA, uPAR, suPA, R2, R3, R4, R5 antibodies for 20 min at 37°C prior to addition of stimuli. U1 parental cell line U937 was used as a control in the transfection experiments. U937 cells were purchased from ATCC and maintained in RPMI 1640 medium in the presence of FCS before and after infection with the X4 HIV-1_{LAI/IIIB}. For infection, cells were seeded at 2×10⁵ cells/ml in 96-well flat bottomed plates (Falcon, Becton-Dickinson Labware, Lincoln Park, NJ) and infected with 1-2×10⁷ virus particles diluted from a stock of pelleted virus (Advanced Biotechnology, Inc., Columbia, MD). After infection, 2 h at 37°C, cells were washed and resuspended in RPMI 1640 medium.

Viral expression was monitored by determination of Mg²⁺-dependent Reverse Trascriptase (RT) activity in culture supernatants (Poli, 1990). In particular, 5 microliters of supernatants was added to 25 microliters of a mixture containing poly(A), oligo(dT) (Pharmacia Fine Chemicals, Piscataway, NJ), MgCl2, and 32P-labeled deoxythymidine 5'-triphosphate (dTTP) (Amersham Corp., Arlington Heights, IL), and incubated for 2 h at 37C. 6 microliters of the mixture was spotted onto DE81 paper, air dried, washed five times in 2x SSC buffer, and two additional times in 95% ethanol. The paper was then dried and counted on a scintillation counter (LS 5000, Beckman Instruments, Inc., Fullerton, CA).

### Viruses

Two R5 HIV-1 strains were used in this study, ADA and Bal. Both HIV-1 stocks were prepared in primary PBMC, supernatants were filtered, 0.2 µm, aliquoted and stored at -80°C.

### Preparation of primary cells and infection conditions

PBMC were obtained from Ficoll-Hypaque (Amersham Pharmacia, Uppsala, Sweden) separated blood of seronegative donors, resupended in RPMI 1640 containing 10% FCS and allowed to activate by PHA (5 micrograms per ml) (Sigma, Milan, Italy) or IL-2 (20 Units per ml) (Chiron, Emeryville, CA). After 3 days cells were washed and resuspended in medium containing IL-2.

MDM cells: PBMC were separated onto an isoosmotic Percoll gradient (Amersham Pharmacia). Purity was consitently >90%, as determined by FACS analysis of CD 14 expression. Monocytes were seeded in 48-well plastic plates (Falcon, Becton Dickinson Labware) at one milion per ml in DMEM (BioWhittaker) supplemented with 10% FCS (Hyclone) and 5% pooled human serum, and allowed to differentiate for 5-7 days. Media and sera were monitored for low content of endotoxin by the Limulus amoebocyte lysate assay (BioWhittaker).

Before infection cells were pretreated with proUPA (at 37°C and 5% CO2) for 10 minutes, at the indicated concentration. Primary cells were infected with 0,1 MOI of an R5 HIV strain. After two hours of adsorption, at 37°C and 5% CO2, virus was extensively washed out and medium containing new aliquot of proUPA was added. Fifty percent of culture medium was changed every 3 days and new medium containing proUPA was added back.

### Measurement of uPAR antigen

Cells were harvested by centrifugation, washed twice with PBS, and lysed in PBS containing 1% Triton X-100. The total protein concentration was determined using a kit (Dc Protein Assay, BIO-RAD) with BSA as standard, and concentration of uPAR antigen by a commercial uPAR ELISA kit (UR-1, Monozyme, Denmark).

### Statistical Analysis

For all experiments, culture supernatants were collected every day for five days, in the case of cell line, or every three days for infection of U937 and primary cells. Reagents were replenished every time culture medium was changed. Data representative of one out of three independent experiments are shown. Results are presented as means of triplicate cultures, and the error bar show the standard deviation from the mean.

### Results

### a. Pro-uPA and ATF inhibit HIV-1 expression in PMA and TNFalpha, but not IL-6, stimulated U1 cells.

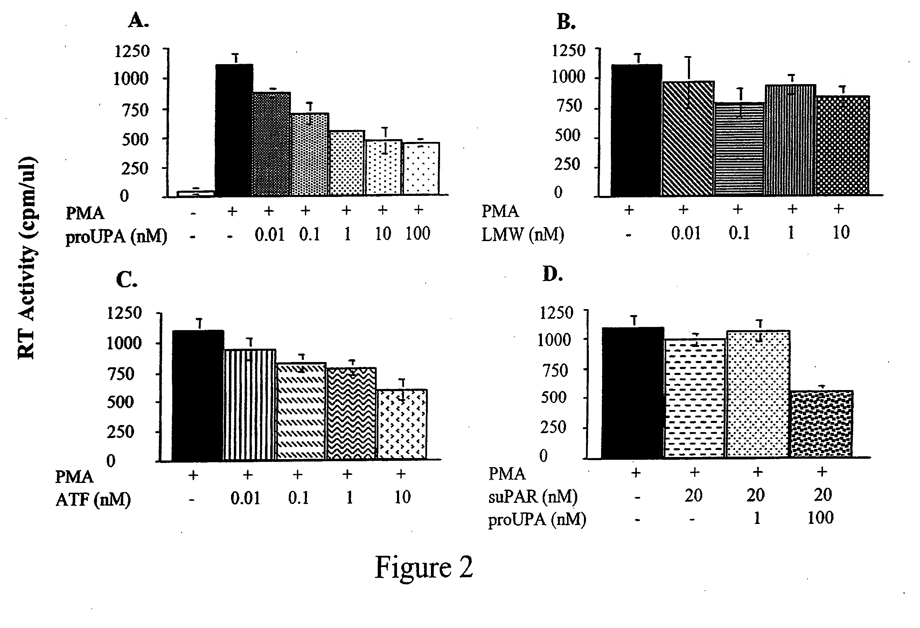

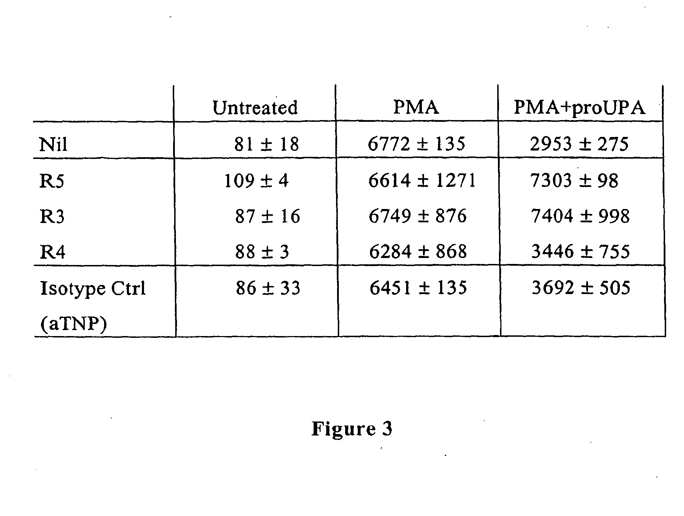

We have tested whether pro-uPA interferes with the induction of HIV-1 by various cytokines. As shown in Figure 2A, treatment with pro-uPA causes a concentration-dependent decrease of PMA-induced HIV-1 replication as measured by RT activity. Inhibition is not complete, reaching about 60% at 1-10 nM pro-uPA. The effect of pro-uPA is specific as can be blocked by a recombinant suPAR (soluble uPAR) which specifically binds pro-uPA preventing its association to the cell surface uPAR (Figure 2D). The effect of pro-uPA might be due to its conversion to uPA and subsequent plasmin formation from serum plasminogen. Alternatively, it might act by binding to uPAR inducing a signal-transduction pathway in competition with PMA. The inhibition of pro-uPA effect by suPAR suggests the latter possibility, suPAR acting as a pro-uPA scavenger, since the pro-uPA-suPAR complex is still enzymatically active but unable to bind cell surface uPAR. To confirm this possibility, we tested both LMW uPA and ATF. LMW-uPA is the carboxyl-terminal domain of uPA with full enzymatic activity and no receptor binding affinity. ATF is the amino terminal fragment of uPA with full receptor-binding and no enzymatic activity (Stoppelli, 1985). As shown in Figure 2B, LMW uPA does not inhibit PMA-induced HIV-1 expression. On the other hand, ATF fully reproduces the pro-uPA effect (Figure 2C). Overall, these data suggest that pro-uPA inhibition of PMA-induced HIV-1 expression requires an interaction between pro-uPA and its cell surface receptor, uPAR. In agreement with this possibility, anti-uPAR antibodies (R3, R5) that prevent the pro-uPA/uPAR interaction also inhibit the effect of pro-uPA (Figure 3). On the other hand the antibody R4, which does not interfere with pro-uPA binding, has no effect. We conclude, therefore, that the inhibition of HIV-1 replication by pro-uPA requires an interaction with uPAR.

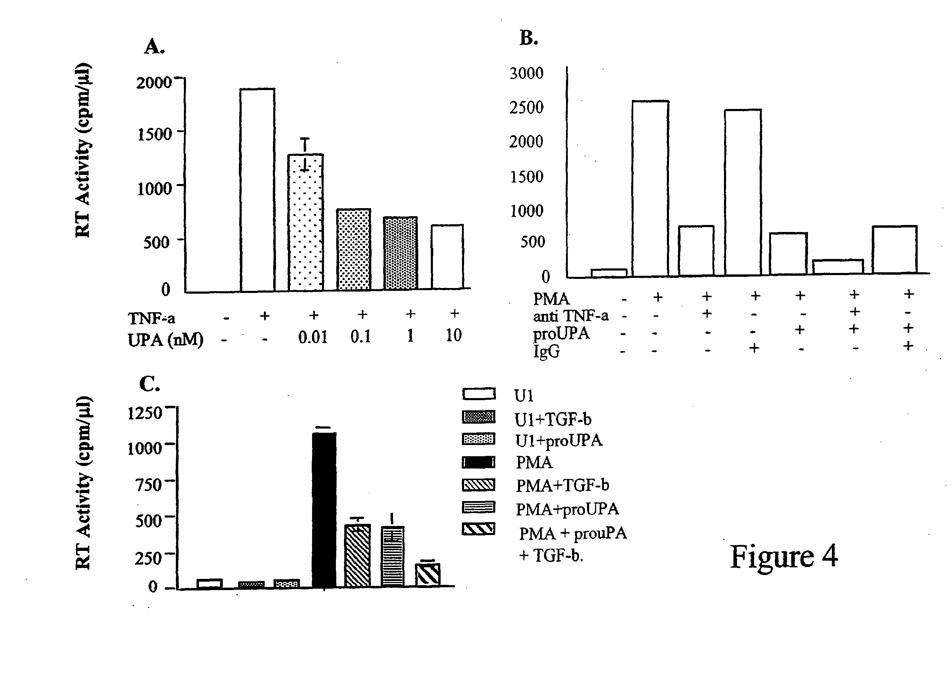

High levels of virus production can be obtained in U1 cells also by stimulation with the pro-inflammatory cytokines TNF-alpha and IL-6. These agents activate HIV-1 expression by activating transcription through NF-kB-dependent (TNF-alpha) or post-transcriptional mechanisms (IL-6) (Duh et al., 1989; Osborn et al., 1989; Poli et al., 1990). UPA (and pro-uPA) is also able to inhibit TNF-α-induced HIV replication (Figure 4A). Since the effect of PMA on HIV-1 replication is in part dependent on the induction of TNF-alpha secretion, and hence partly inhibited by an anti-TNF-alpha antibody (Poli et al., 1990), one would expect the combination of the anti-TNF-alpha antibody with pro-uPA to be a more potent inhibitor of PMA-induced HIV-1 replication/expression. As shown in Figure 4B, while anti-TNF-alpha and pro-uPA individually inhibit HIV-1 replication about 70%, together the reach a bigger effect, inhibiting by over 90%. This result suggests that pro-uPA and TNF-alpha act on a parallel signaling pathway.

TGF-β also inhibits PMA-induced HIV-1 replication/expression (Poli et al., 1991) in a NF*-k*B independent way. As shown in Figure 4C, addition of the TGF-β and pro-uPA combination results in an additive inhibitory effect, about 90% inhibition with respect to the 60% obtained with only one reagent. These results therefore strongly indicate that pro-uPA interferes with a signaling step different from those activated by TGF-β or TNF-α and indicate that these steps are activated.

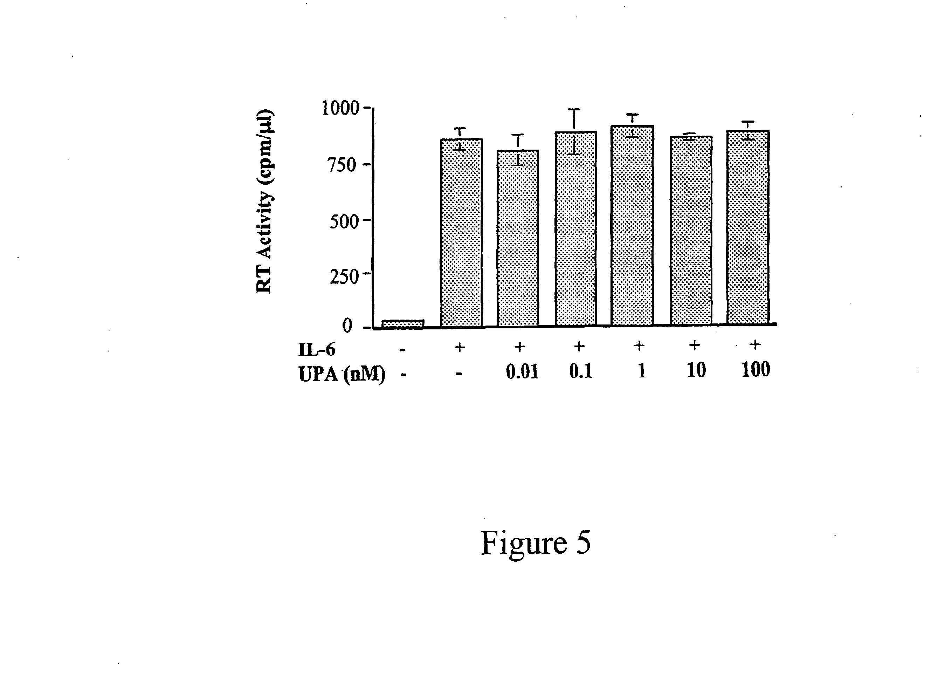

Unlike for PMA and TNF-α pro-uPA has no effect on the IL-6 dependent HIV-1 replication in U1 cells (Figure 5). Overall the results obtained with different citokines, suggest that pro-uPA acts by interfering with one specific signaling pathways that can lead to the inhibition of virus expression.

### b. U1 cells express cell surface uPAR and shed it to the conditioned medium.

Since the inhibition by pro-uPA of HIV-1 replication requires an interaction with uPAR, we have tested whether U1 cells express uPAR. The test was performed by an immuno-blot analysis of lysates of U1 cells, demonstrating the presence of a specific band reacting with anti-uPAR monoclonal antibodies. A similar analysis of the conditioned media shows the presence of uPAR in both untreated and PMA-treated U1 cells, in which uPAR is increased. Measurement of the amount of uPAR by ELISA gave essentially the same results (data not shown). These data are in agreement with previous reports showing that PMA can induce uPAR in U937 cells (Picone, 1989).

### c. proUPA affects HIV-1 replications in primary cells.

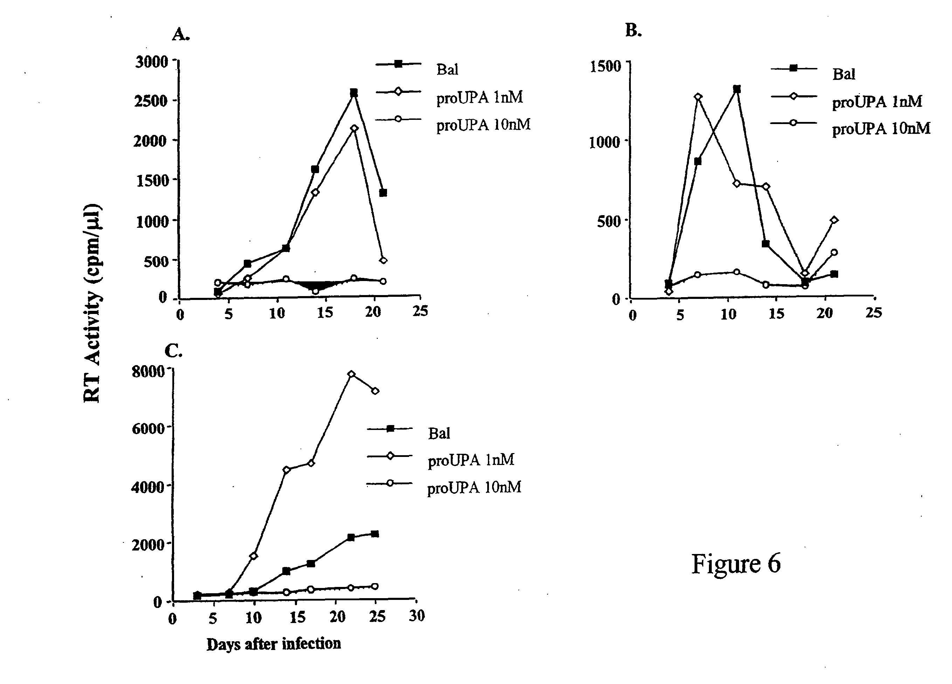

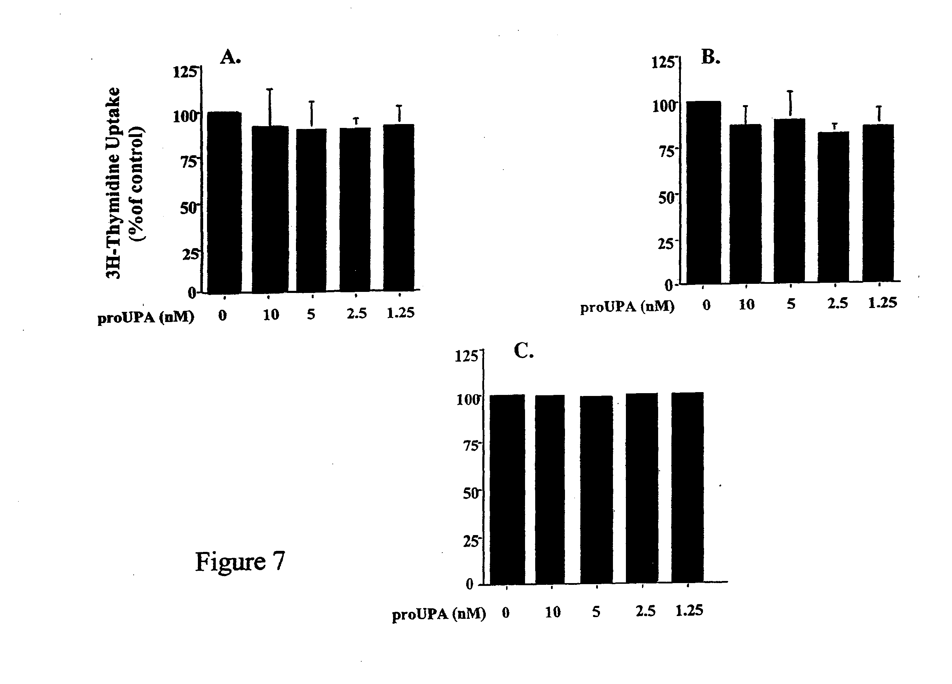

When assayed on human peripheral blood monocytic cells (PBMC), pro-uPA was found to also affect the replication of HIV. However, qualitatively the effect of pro-uPA was shown to be dependent on its concentration and on the nature of the cells. In fact in the PHA-stimulated PBMC and IL-2 stimulated PBMC we found no effect of pro-uPA at 1 nM and a large inhibition of virus growth at 10 nM (Figure 6A and B). With monocytes derived macrophages, on the other hand, low concentrations of pro-uPA (10 nM) stimulated virus expression while higher concentrations inhibited it (Figure 6C). Pro-uPA was not toxic to the cells: at 10 nM pro-uPA it showed essentially no effect on cellular proliferation as measured by ³H-thymidine uptake in uninfected PHA-stimulated PBMC, virus infected PHA-stimulated PBMC nor in uninfected resting PBMC (Figure 7A, B, C).

### d. Correlation between viral replication and uPAR expression level.

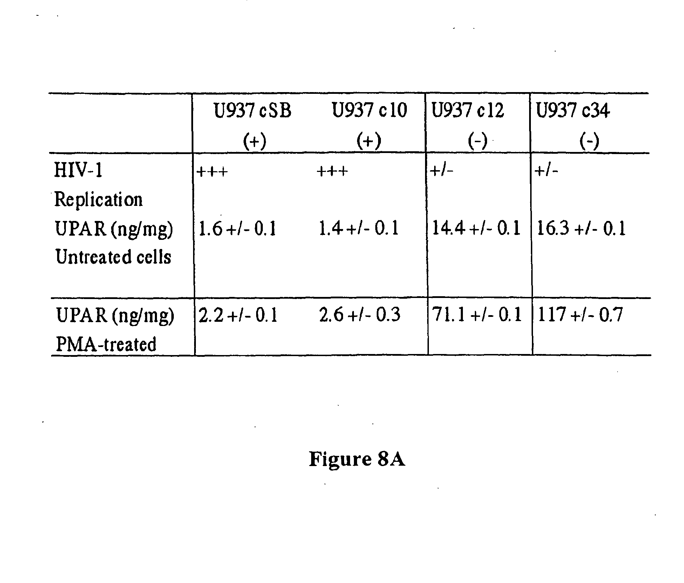

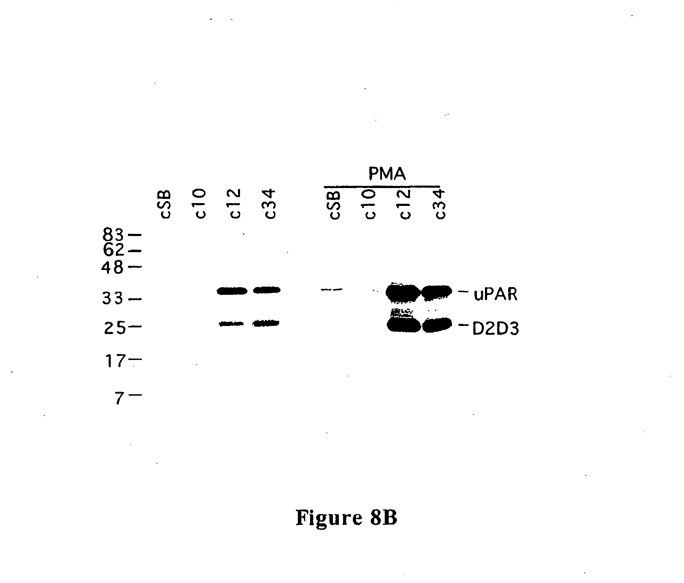

We compared the levels of uPAR in various clones of U937 cells distinguished by their high ("plus" clones) or low efficiency ("minus" clones) in supporting HIV replication. Figure 8A shows a correlation between the ability of HIV to replicate and the levels of uPAR measured by an ELISA. Non stimulated clones of U937 cells capable of sustaining productive HIV infection ("plus" clones: cSB and c10) expressed around 10-fold less uPAR than did U937 clones displaying delayed and/or reduced productive HIV infection ("minus" clones: c12 and c34). This difference was even bigger (around 30-fold) when cells were stimulated for 24 hours with PMA, since the "plus" clones were unable to up-regulate uPAR. Figure 8B shows an additional approach to compare the levels of uPAR in the minus and plus clones, performed by immunoblotting of equal amounts of proteins from lysates of two clones each. This example clearly demonstrates that productive HIV infection of U937 cells inversely correlates with the expression level of uPAR. Indeed, cell clones able to sustain a high efficiency of viral replication have a very low uPAR level and do not even increase uPAR expression under conditions (treatment with PMA) in which uPAR is normally induced several fold in the same cell line (Picone, 1989).

### EXAMPLE 2

### Specific antibodies to detect and quantitate "activated" uPAR fragments.

The chemotactic D2D3₈₈₋₂₇₉ fragment of uPAR is generated in vitro by cleavage with chymotrypsin of a soluble form of uPAR (Fazioli, 1997) (see Figure 1). A form of uPAR containing domains D2 and D3 and starting at residue 84 has been identified in vivo: in vitro, uPA itself at physiological concentrations cleaves suPAR and cell surface uPAR at residue 84 (Hoyer-Hansen, 1997; Hoyer-Hansen, 1992; Sidenius, 2000). In addition, the synthetic uPAR peptide with the highest chemotactic activity encompasses residues 84-97 (Fazioli, 1997). It is possible therefore that the activation of uPAR in tissues is due to cleavage by uPA. Finally, the failure of uPA-deficient mice to cleave uPAR (data not shown) suggests that the migration-defective phenotype is due to the lack of cleavage.

Since uPAR cleavage can "activate" uPAR conferring a migration-promoting phenotype, cleavage of uPAR therefore must confer an "inflammatory" phenotype to cells. However, cleavage in the linker region between domain D1 and D2 would have very different effects depending on where the cleavage occurs. For example, cleavage between residues 84 to 89 would generate an "activated" uPAR, while cleavage between residue 89 through 93 would destroy the activity. This is based on the finding that while peptides AVTYSRSRYLEC and SRSRY are active in chemotaxis-promotion, cleavage of the AVTYSRSRYLEC peptide with plasmin (which cleave immediately after the two arginine residues) destroyed the activity (data not shown). Clearly, the detection of the presence of "activated" uPAR and its quantititive measurement (in blood, urine, cells or tissue fragments) with a simple assaya would be useful in evaluating the inflammatory state of an individual. However, current methods do not allow an easy evaluation of where uPAR is cleaved as they require protein purification and sequencing.

Antibodies, however, can discriminate between very similar amino acids sequences or even sequences differing by one single residue. We have searched, therefore, for antibodies specific for "activated" uPAR fragments.

### Materials and methods

### Preparation of peptide 3 antiserum

Peptide 3 (AVTYSRSRYLEC) (Blasi, 1997) was synthesized, coupled to keyhole limpet hemacyanin (KLH), and a rabbit antiserum generated by standard methods. The peptide-3 specific immunoglobulins were purified by affinity chromatography on peptide 3-Sepharose columns, by standard methods.

### Expression and purification of recombinant uPAR and uPAR fragments

Full-length soluble uPAR (suPAR) was expressed in CHO cells stably transfected with a cDNA encoding aminoacid 1-278 of mature human uPAR and purified by affinity-chromatography on a column with R2 monoclonal antibody immobilized on CNBr activated Sepharose (Pharmacia) as described (Blasi, 1997; Fazioli, 1997). We have also employed similar recombinant fragments containing at their carboxy terminus the FLAG epitope (Prickett, 1989). D2D3₈₈₋₂₇₈ and D2D3₈₇₋₂₇₈ were prepared by hydrolysis of full-length suPAR with chymotrypsin and MMP-12, respectively, followed by purification of the D2D3 moiety on the same column as described above. The recombinant proteins D2D3₈₄₋₂₇₈/_{FLAG} and D2D3₉₂₋₂₇₈/_{FLAG} were purified from the conditioned medium of transiently transfected COS7 cells and purified on a M2-affinity column (Sigma) according to the manufactures instruction.

### Western blotting assays

10 ng of purified suPAR, D2D3₈₈₋₂₇₈, D2D3_{84-278/FLAG} and D2D3_{92-278/FLAG} proteins were denatured under lightly reducing conditions and enzymatically deglycosylated using PNGase F (Boehringer) as described elsewhere (Dano, 1990). The deglycosylated proteins were separated by 15% SDS-PAGE and transferred to PVDF membranes using semi-dry electro transfer. After blocking in 5% non-fat dry milk, blots were incubated with 0.5 µg/ml of a polyclonal anti-uPAR antibody recognizing all known forms of uPAR or with a 1:2000 dilution of the anti-Pep3 serum. The blots were washed, incubated with a secondary horse-radish peroxidase conjugated donkey-anti-rabbit antibody (1:5000, Amersham), washed, and developed using a chemoluminescent substrate (SuperSignal Dura, Pierce).

### ELISA assay

NUNC maxisorb 96-well plates were coated ON with 1 µg/ml R2 antibody diluted in coating buffer (0.1 M NaHPO4, pH 9.6). The plates were washed three times with PBS-T (PBS containing 0.1% Tween 20) and blocked for one hour at 37°C with 2% BSA in PBS. Separate wells were incubated with the recombinant purified suPAR and D2D3 fragments generated by cleavage with chymotrypsin or MMP-12, and diluted to 10 ng/ml in dilution buffer (PBS containing 1% BSA). After incubation at 37°C for one hour the plates were washed and incubated for one hour at 37°C with either a polyclonal anti-uPAR antibody recognizing all forms of uPAR or the affinity purified anti-peptide 3 antibody, both diluted to 1 µg/ml in dilution buffer. After additional washing the plates were probed for one hour at 37°C with alkaline phosphatase conjugated mouse-anti-rabbit antibody (1:1000 Sigma), washed and developed with a chromogenic alkaline phosphatase substrate (pNPP, Sigma) as recommended by the manufacturer. Specific binding was determined by subtracting the absorbance observed in well receiving no suPAR or suPAR fragments. The data are presented as the average (+/- SD) of a duplicate determination.

### Results

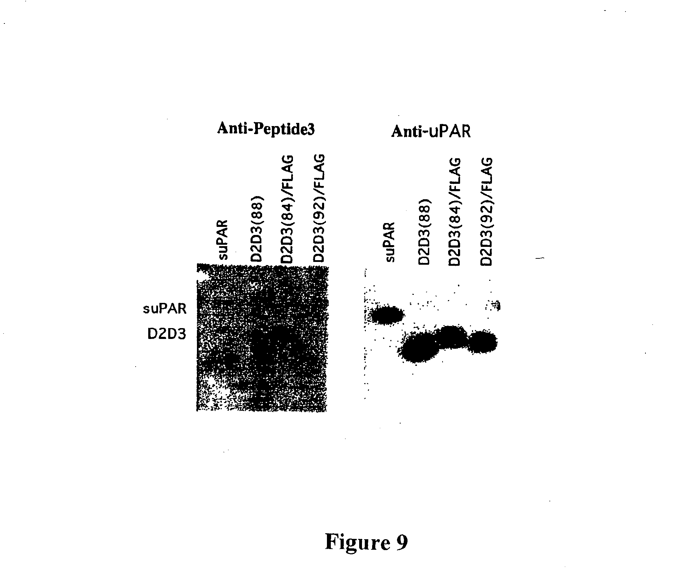

To determine the specificity of the Peptide3 antibody, western blotting experiments were performed on suPAR and D2D3 fragments carrying different well-defined parts of the uPAR linker region. When blots were probed with the anti-Peptide3 antibody the D2D3₈₄₋₂₇₈/_{FLAG}, but not the suPAR, D2D3₈₈₋₂₇₈, or D2D3_{92-278/FLAG} proteins, was efficiently recognized (Figure 9, left panel). This was not caused by different loading of the proteins on the gel as all recombinant proteins were equally well recognized by a polyclonal antibody directed against intact suPAR (Figure 9, right panel). Surprisingly, the anti-Peptide 3 antibody did not recognize intact suPAR (which obviously carries the entire linker region). This experiment demonstrates that the anti-peptide 3 antibody preferentially recognizes cleaved uPAR and that the epitope(s) recognized includes one or more of the aminoacids Ala84-Tyr87. It also suggests that the sequence 85-87 is buried in the intact uPAR and becomes exposed when uPAR is cleaved.

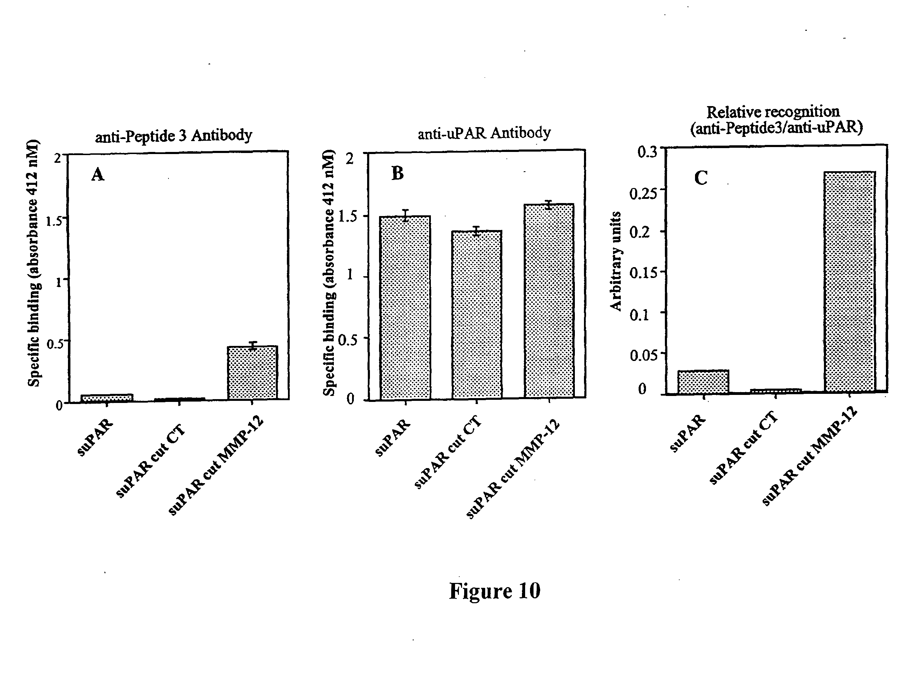

These observations were confirmed using an independent in vitro binding (ELISA) experiment. In this assay suPAR and the different D2D3 proteins are first captured using a monoclonal antibody (R2, which recognizes the carboxy termini of all suPAR forms, cleaved by a protease in vitro) previously adsorbed to plastic. In a second step the bound proteins were probed with the polyclonal (rabbit) antiserum directed against Peptide 3 or against intact uPAR. The bound rabbit antibodies were quantified using anti-rabbit enzyme-conjugated secondary antibody and a colorimetric detection. While the anti-uPAR antiserum recognized equally suPAR, chymotrypsin-cleaved suPAR and MMP-12-cleaved suPAR (Figure 10, panel B), the anti-peptide-3 IgG only recognized an MMP-12-cleaved suPAR (Figure 10, panel A). Chymotrypsin cleaves uPAR at residue 87-88(Behrendt, 1991), while MMP-12 cleaves at residue 86/87 (data not shown). Therefore, the anti-peptide 3 IgG specifically recognize D2D3 fragments whose amino terminus carries at least residue 84, or residues 84-85 or residues 84-87 of uPAR, demonstrating therefore high discriminating ability among D2D3 fragments. When the data obtained with anti-peptide 3 and anti-uPAR antibodies were divided by one another, the ratio anti-peptide-3/anti-whole uPAR was 0.024 with suPAR and 0.005 with chymotrypsin-cleaved suPAR. However,the ratio was 0.27 with MMP-12-cleaved suPAR (Figure 10, panel C).

Overall the data demonstrate that the aminoterminal border of the epitope recognized by the anti-Peptide3 antibody is Tyr87 as D2D3 fragments generated by MMP12 (having Tyr87 as N-terminal aminoacid) are fully recognized, while D2D3 fragment generated by chymotrypsin (having Ser88 as N-terminal aminoacid) are not. This experiment also demonstrates that a simple ELISA can be used to selectively detect specific forms of cleaved uPAR.

### REFERENCES QUOTED

Aguirre Ghiso, J. A., Kovalski, K., and Ossowski, L. (1999). Tumor dormancy induced by downregulation of urokinase receptor in human carcinoma involves integrin and MAPK signaling. J Cell Biol *147,* 89-104.

B. Degryse, M. R., S. Rabbani, A. Villa, F. Fazioli and Blasi, F. (1999). Src-dependence and pertussis-toxin sensitivity of urokinase receptor-dependent chemotaxis, and cytoskeleton reorganization in rat smooth muscle cells via the urokinase receptor. Blood ***94,*** 649-662.

Behrendt, N., Ploug, M., Patthy, L., Houen, G., Blasi, F. & Danø, K. (1991). The ligand-binding domain of the cell surface receptor for urokinase-type plasminogen activator. J. Biol. Chem. ***266,*** 7842-7847.

Behrendt, N., Jensen, O.N., Engelholm, L.H., Moertz, E., Mann, M. and Dan, K. (2000). A urokinase receptor-associated protein with specific collagen binding properties. J. Biol. Chem. *275,* 1993-2002.

Blasi, F., Fazioli, F., Resnati, M., Sidenius,N. (1997). UPAR mimicking peptide. In WO 98/42733, priority 20-March-97.

Blasi, F. (1997). uPAR-uPA-PAI-1: a key intersection in proteolysis, adhesion and chemotaxis. Immunol. Today ***18***, 415-417.

Blasi, F., Conese, M., Møller, L.B., Pedersen, N., Cavallaro, U., Cubellis, M.V., Fazioli; F., Hernandez-Marrero, L., Limongi, P., 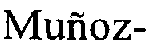 Canoves, P., Resnati, M., Riittinen, L., Sidenius, N., Soravia, E., Soria, M.R., Stoppelli, M.P., Talarico, D., Teesalu, T., Val (1994). The urokinase receptor: structure, regulation and inhibitor-mediated internalization. *Fibrinolysis **8** (Suppl. 1*), 182-188.

Borghi, M. O., Panzeri, P., Shattock, R., Sozzani, S., Dobrina, A., and Meroni, P. L. (2000). Interaction between chronically HIV-infected promonocytic cells and human umbilical vein endothelial cells: role of proinflammatory cytokines and chemokines in viral expression modulation. Clin Exp Immunol *120*, 93-100.

Boyle, M. D. P., Chiodo, V.A., Lawman, M.J.P., Gee, A.P. and Young, M. (1987). Urokinase: a chemotactic factor for polymorphonuclear leukocytes in vivo. J. Immunol. *139*, 169-174.

Butera, S. T., Roberts, B. D., Lam, L., Hodge, T., and Folks, T. M. (1994). Human immunodeficiency virus type 1 RNA expression by four chronically infected cell lines indicates multiple mechanisms of latency. J Virol *68*, 2726-30.

Cannon, P., Kim, S. H., Ulich, C., and Kim, S. (1994). Analysis of Tat function in human immunodeficiency virus type 1-infected low-level-expression cell lines U1 and ACH-2. J Virol *68,* 1993-7.

Chapman, H. A. (1997). Plasminogen activators, integrins, and the coordinated regulation of cell adhesion and migration. Curr. Opin. Cell Biol. *9*, 714-724.

Coleman JL, G. J., Benach JL (2001). Borrelia burgdorferi and other bacterial products induce expression and release of the urokinase receptor. J. Immunol. *166*, 473-480.

Colman, R. W., Pixley, R. A., Najamunnisa, S., Yan, W., Wang, J., Mazar, A., and McCrae, K. R. (1997). Binding of high molecular weight kininogen to human endothelial cells is mediated via a site within domains 2 and 3 of the urokinase receptor. J Clin Invest *100,* 1481-7.

Dano, K., Behrendt, N., Brunner, N., Ellis, V., Plough, M. and Pyke, C. (1994). The urokinase receptor: protein structure and role in plasminogen activation and cancer invasion. Fibrinolysis **8** (suppl. 1), 189-203.

Dano, K., Blasi, F. et al. (1990). Urokinase-type plasminogen activator receptor. In WO90/12091.

Degryse, B., Resnati, M., Rabbani, S. A., Villa, A., Fazioli, F., & Blasi, F. (1999). Src-dependence and pertussis-toxin sensitivity of urokinase receptor-dependent chemotaxis, and cytoskeleton reorganization in rat smooth muscle cells via the urokinase receptor. Blood *94*, 649-662.

Degryse, B., Orlando, S., Resnati, M., Rabbani, S.A. and Blasi, F. (2001). Urokinase/urokinase receptor and vitronectin/avb3 integrin induce chemotaxis and cytoskeleton reorganization through different signaling pathways. Oncogene *in press.*

Deng, G., Curriden, S. A., Wang, S., Rosenberg, S., and Loskutoff, D. J. (1996). Is plasminogen activator inhibitor-1 the molecular switch that governs urokinase receptor-mediated cell adhesion and release? J Cell Biol *134,* 1563-71.

Duh, E. J., Maury, W. J., Folks, T. M., Fauci, A. S., and Rabson, A. B. (1989). Tumor necrosis factor alpha activates human immunodeficiency virus type 1 through induction of nuclear factor binding to the NF-kappa B sites in the long terminal repeat. Proc Natl Acad Sci U S A *86*, 5974-8.

Emiliani, S., Van Lint, C., Fischle, W., Paras, P., Jr., Ott, M., Brady, J., and Verdin, E. (1996). A point mutation in the HIV-1 Tat responsive element is associated with postintegration latency. Proc Natl Acad Sci U S A *93*, 6377-81.

Fazioli, F., Resnati, M, Sidenius, N, Higashimoto, Y, Appella, E and Blasi, F. (1997). The urokinase-sensitive region of the urokinase receptor is responsible for its potent chemotactic activity. EMBO J. *16,* 7279-7286.

Fazioli, F. and Blasi, F. (1994). Urokinase-type plasminogen activator and its receptor: new target for anti-metastatic therapy? TiPS *15,* 25-29.

Fibbi, G., Ziche, M., Morbidelli, L., Magnelli, L. and Del Rosso, M. (1988). Interaction of urokinase with specific receptors stimulates mobilization of bovine adrenal capillary endothelial cells. Exp. Cell Res. *179,* 385-395.

Finzi, D., Blankson, J., Siliciano, J. D., Margolick, J. B., Chadwick, K., Pierson, T., Smith, K., Lisziewicz, J., Lori, F., Flexner, C., Quinn, T. C., Chaisson, R. E., Rosenberg, E., Walker, B., Gange, S., Gallant, J., and Siliciano, R. F. (1999). Latent infection of CD4+ T cells provides a mechanism for lifelong persistence of HIV-1, even in patients on effective combination therapy [see comments]. Nat Med *5*, 512-7.

Folks, T. M., Justement, J., Kinter, A., Dinarello, C. A., and Fauci, A. S. (1987). Cytokine-induced expression of HIV-1 in a chronically infected promonocyte cell line. Science *238*, 800-2.

Folks, T. M., Justement, J., Kinter, A., Schnittman, S., Orenstein, J., Poli, G., and Fauci, A. S. (1988). Characterization of a promonocyte clone chronically infected with HIV and inducible by 13-phorbol-12-myristate acetate. J Immunol *140,* 1117-22.

Franzoso, G., Biswas, P., Poli, G., Carlson, L. M., Brown, K. D., Tomita-Yamaguchi, M., Fauci, A. S., and Siebenlist, U. K. (1994). A family of serine proteases expressed exclusively in myelo-monocytic cells specifically processes the nuclear factor-kappa B subunit p65 in vitro and may impair human immunodeficiency virus replication in these cells. J Exp Med *180,* 1445-56.

Gudewicz, P. W. a. B., N. (1987). Human urokinase-type plasminogen activator stimulates chemotaxis of human neutrophils. Biochem. Biophys. Res. Comm., *147*, 1176-1181.

Gyetko, M. R., Chen, G.-H., McDonald, R.A., Goodman, R., Huffnagle, G.B., Wilkinson, C.C., Fuller, J.A. and Toews, G.B. (1996). Urokinase is required for the pulmonary inflammatory response to Cryptococcus neoformans. A murine transgenic model. J. Clin. Invest. *97*, 1818-1826.

Gyetko, M. R., Todd, R. F. 3rd, Wilkinson, C. C., and Sitrin, R. G. (1994). The urokinase receptor is required for human monocyte chemotaxis in vitro. J. Clin. Invest. *93*, 1380-1387.

Gyetko, M. R., Sud, S., Kendall, T., Fuller, J.A., Newstead, M.W., Standiford, T.J. (2000). Urokinase receptor-deficient mice have impaired neutrophil recruitment in response to pulmonary Pseudomonas aeruginosa infection. J. Immunol. *165,* 1513-1519.

Handley, M. A., Steigbigel, R.T. and Morrison, S.A. (1996). A role for urokinase-type plasminogen activator in human immunodeficiency virus type 1 infection of macrophages. J. Virol. *70*, 4451-4456.

Hoyer-Hansen, G., Ploug, M., Behrendt, N., Ronne, E. and Dano, K. (1997). Cell surface acceleration of urokinase-catalyzed receptor cleavage. Eur J Biochem *243,* 21-26.

Hoyer-Hansen, G., Behrendt, N., Ploug, M., Dano, K. and Preissner, K. T. (1997). The intact urokinase receptor is required for efficient vitronectin binding: receptor cleavage prevents ligand interaction. FEBS Lett *420*, 79-85.

Hoyer-Hansen, G., Ronne, E., Solberg, H., Behrendt, N., Ploug, M., Lund, L.R., Ellis, V. and Dano, K. (1992). Urokinase plasminogen activator cleaves its cell surface receptor releasing the ligand-binding domain. J Biol Chem *267*, 18224-18229.

Mustjoki, S., Sidenius, N. Sier, C.F.M. Blasi, F. Elonen, E., Alitalo, R. and Vaheri, A. (2000). Levels of soluble urokinase receptor correlate with number of circulating tumor cells in acute leukemia and decrease rapidly during chemotherapy. Cancer Res. *60*, 7126-7132.

Muto, N. F., Martinand-Mari, C., Adelson, M. E., and Suhadolnik, R. J. (1999). Inhibition of replication of reactivated human immunodeficiency virus type 1 (HIV-1) in latently infected U1 cells transduced with an HIV-1 long terminal repeat-driven PKR cDNA construct. J Virol *73*, 9021-8.

Nguyen, D. H., Webb, D.J., Catling, A.D., Song, Q., Dhakephalkar, A., Weber, M.J., Ravichandran, K.S. and Gonias, S.L. (2000). Urokinase-type plasminogen activator stimulates the Ras/Extracellular signal-regulated kinase (ERK) signaling pathway andMCF-7 cell migration by a mechanism that requires focal adhesion kinase, Src, and Shc. Rapid dissociation of GRB2/Sps-Shc complex is associated with the transient phosphorylation of ERK in urokinase-treated cells. J. Biol. Chem. 275, 19382-19388.

Nykjaer, A., Christensen, E.I., Vorum, H., Hager, H., Petersen, C.M., Roigaard, H., Min, H.Y., Vilhardt, F., Moller, L.B., Kornfeld, S. and Gliemann, J. (1998). Mannose-6-phosphate/insulin-like growth factor-II receptor targets the urokinase receptor to lysosomes via a novel binding interaction. J. Cell Biol. *141,* 815-828.

Nykjær, A., Møller, B., Todd III, R.F., Christensen, T., Andreasen, P.A., Gliemann, J. and Petersen, C.M. (1994). Urokinase receptor. An activation antigen in human T lymphocytes. *J. Immunol.* ***152,*** 505-516.

Osborn, L., Kunkel, S., and Nabel, G. J. (1989). Tumor necrosis factor alpha and interleukin 1 stimulate the human immunodeficiency virus enhancer by activation of the nuclear factor kappa B. Proc Natl Acad Sci U S A *86*, 2336-40.

Ossowski, L., Aguirre Ghiso, J. A. (2000). Urokinase receptor and integrin partnership: coordination of signaling for cell adhesion, migration and growth. Curr. Opin. Cell Biol. *12*, 613-620.

Pedersen, N., Schmitt, M., Rønne, E., Nicoletti, M.I. Høyer-Hansen, G., Conese, M., Giavazzi, R., Danø, K., Kuhn, W. Jänicke, F. and Blasi, F. (1993). An unoccupied, water soluble urokinase receptor is present in the ascitic fluid and plasma from patients with ovarian cancer. J. Clin. Invest. ***92,*** 2160-2167.

Picone, R., Kajtaniak, E.L., Nielsen, L. S., Behrendt, N., Mastronicola, M. R., Cubellis, M.V., Stoppelli, M.P., Pedersen, S., Danø, K. and Blasi, F. (1989). Regulation of urokinase receptors in monocyte-like U937 cells by phorbol esters phorbol myristate acetate. *J. Cell Biol.* ***108,*** 693-702.

Poli, G., Bressler, P., Kinter, A., Duh, E., Timmer, W. C., Rabson, A., Justement, J. S., Stanley, S. and Fauci, A. S. (1990). Interleukin 6 induces human immunodeficiency virus expression in infected monocytic cells alone and in synergy with tumor necrosis factor alpha by transcriptional and post-transcriptional mechanisms. J Exp Med *172*, 151-8.

Poli, G., Kinter, A., Justement, J. S., Kehrl, J. H., Bressler, P., Stanley, S., and Fauci, A. S. (1990). Tumor necrosis factor alpha functions in an autocrine manner in the induction of human immunodeficiency virus expression. Proc Natl Acad Sci U S A *87*, 782-5.

Poli, G., Kinter, A. L., Justement, J. S., Bressler, P., Kehrl, J. H., and Fauci, A. S. (1991). Transforming growth factor beta suppresses human immunodeficiency virus expression and replication in infected cells of the monocyte/macrophage lineage. J Exp Med *173*, 589-97.

Pomerantz, R. J., Trono, D., Feinberg, M. B., and Baltimore, D. (1990). Cells nonproductively infected with HIV-1 exhibit an aberrant pattern of viral RNA expression: a molecular model for latency. Cell *61,* 1271-6.

Resnati, M., Guttinger, M., Valcamonica, S., Sidenius, N., Blasi, F. and Fazioli, F. (1996). Proteolytic cleavage of the urokinase receptor substitutes for the agonist-induced chemotactic effect. *EMBO J. 15,* 1572-1582.

Ronne, E., Behrendt, N., Ellis, V., Ploug, M., Danø, K. and Hoyer-Hansen, G. (1991). Cell induced potentiation of the plasminogen activation system is abolished by a monoclonal antibody that recognizes the N-terminal domain of the urokinase receptor. *FEBS Lett.* ***288,*** 233-236.

Sidenius, N., Olsen, J.E., Sier, C.F.M. Blasi, F., Ullum, H. (2000). Increased plasma levels of soluble urokinase receptor in HIV infected patients are highly correlated with survival. Blood *96*, 4091-4095.

Sidenius, N., Nicoletti, I., Mariani, A., Aletti, G., Frigerio, L., Ferrari, A., Agape, V., Stephens, R. W., Frandsen, T.L., Brünner, N., Giavazzi, R., Blasi, F. and Sier, C.F.M. (2001). Presence of domain 1 fragment of urokinase-type plasminogen activator receptor (uPAR) in human body fluids. Submitted.

Sidenius, N., Sier, C F.M. and Blasi, F. (2000). Shedding and cleavage of the urokinase receptor (uPAR): Identification and characterisation of uPAR fragments *in vitro* and *in vivo.* FEBS L. *475*, 52-56.

Sidenius, N. and Blasi, F. (2000). Domain 1 of the urokinase receptor (uPAR) is required for uPAR-mediated cell binding to vitronectin. FEBS L. *470,* 40-46.

Simon, D. I., Wei, Y., Zhang, L., Rao, N.K., Xu, H., Chen, Z., Liu, Q., Rosenberg, S., Chapman, H.A. (2000). Identification of a urokinase receptor-integrin interaction site. Promiscuous regulator of integrin function. J. Biol. Chem. *275*, 10228-10234.

Slot, O., Brunner, N. and Stephens, R.W. (2000). Marker of erosive progression in RA. Ann. Rheum. Dis. *59*, 656.

Slot, O., Brunner, N., Locht, H., Oxholm, P. and Stephens, R.W. (1999). Soluble urokinase plasminogen activator receptor in plasma of patients with inflammatory rheumatic disorders: increased concentrations in rheumatoid arthritis. Ann. Rheum. Dis. *58,* 488-492.

Solberg, H., Romer, J., Brünner, N., Holm, A., Sidenius, N., Danø, K. and Høyer-Hansen, G. (1994). A cleaved form of the receptor for urokinase-type plasminogen activator in invasive transplanted human and murine tumors. Int J Cancer *58,* 877-881.

Speth, C., Pichler, I., Stockl, G., Mair, M., Dierich, M.P. (1998). Urokinase plasminogen activator receptor (uPAR; CD87) expression on monocytic cells and T cells is modulated by HIV-1 infection. Immunobiology *199*, 152-162.

Stephens, R. W., Nielsen, H.J., Christensen, I.J., Thorlacius-Ussing, O., Sorensen, S., Dano, K., Brunner, N. (1999). Plasma urokinase receptor levels in patients with colorectal cancer: relationship to prognosis. J. Natl Cancer Inst *91,* 869-874.

Stoppelli, M. P., Corti, A., Soffientini, A., Cassani, G., Blasi, F. and Assoian, R.K. (1985). Differentiation-enhanced binding of the amino-terminal fragment of human urokinase plasminogen activator to a specific receptor on U937 monocytes. *Proc. Natl. Acad. Sci. USA **82**,* 4939-4943.

Tarui, T., Mazar, A.P., Cines, D.B., Takada, Y. (2000). Urokinase receptor (uPAR/CD87) is a ligand for integrin and mediates cell-cell interaction. J. Biol. Chem. *In press, MS. M0082202000, October 26.*

Vassalli, J.-D., Baccino, D. and Belin, D. (1985). A cellular binding site for the Mr 55,000 form of the human plasminogen activator urokinase. *J. Cell Biol. **100**,* 86-92.

Vicenzi, E., Biswas, P., Mengozzi, M., and Poli, G. (1997). Role of pro-inflammatory cytokines and beta-chemokines in controlling HIV replication. J Leukoc Biol *62*, 34-40.

Waltz, D. A., and Chapman, H. A. (1994). Reversible cellular adhesion to vitronectin linked to urokinase receptor occupancy. J. Biol. Chem. *269*, 14746-14750.

Waltz, D. A., Natkin, L. R., Fujita, R. M., Wei, Y., and Chapman, H. A. (1997). Plasmin and plasminogen activator inhibitor type 1 promote cellular motility by regulating the interaction between the urokinase receptor and vitronectin. J Clin Invest *100,* 58-67.

Webb, D. J., Nguyen, D. H., and Gonias, S. L. (2000). Extracellular signal-regulated kinase functions in the urokinase receptor-dependent pathway by which neutralization of low density lipoprotein receptor-related protein promotes fibrosarcoma cell migration and Matrigel invasion. J Cell Sci *113,* 123-134.

Wei, Y., Lukashev, M., Simon, D. I., Bodary, S. C., Rosenberg, S., Doyle, M. V., and Chapman, H. A. (1996). Regulation of integrin function by the urokinase receptor. Science *273*, 1551-5.

Wei, Y., Yang, X., Liu, Q., Wilkins, J. A., and Chapman, H. A. (1999). A role for caveolin and the urokinase receptor in integrin-mediated adhesion and signaling. J Cell Biol *144,* 1285-94.

Xue, W., Kindzelskii, A. L., Todd, R. F., 3rd, and Petty, H. R. (1994). Physical association of complement receptor type 3 and urokinase-type plasminogen activator receptor in neutrophil membranes. J Immunol *152*, 4630-40.

Xue, W., Mizukami, I., Todd, R. F., 3rd, and Petty, H. R. (1997). Urokinase-type plasminogen activator receptors associate with beta1 and beta3 integrins of fibrosarcoma cells: dependence on extracellular matrix components. Cancer Res *57*, 1682-9.

Yang, X., Chen, Y., and Gabuzda, D. (1999). ERK MAP kinase links cytokine signals to activation of latent HIV-1 infection by stimulating a cooperative interaction of AP-1 and NF-kappaB. J Biol Chem *274*, 27981-8.

Yebra, M., Parry, G. C. N., Stromblad, S., Mackman, N., Rosenberg, S., Mueller, B. M., and Cheresh, D. A. (1996). Requirement of receptor-bound urokinase-type plasminogen activator for integrin alphavbeta5-directed cell migration. J Biol Chem *271*, 29393-9.

## Claims

1. The use of pro-uPA, a derivative or fragment thereof retaining full uPAR binding without the uPA carboxyl-terminal domain carrying the enzymatic activity, or of activated uPAR molecules, for the preparation of a medicament for the treatment of HIV-infection.

2. The use according to claim 1, wherein said pro-uPA derivative or fragment is selected from the group consisting of: a) a mutant of pro-uPA in the active site, b) the pro-uPA amino terminal fragment and c) a fragment comprising the growth-factor domain.

3. The use of pro-uPA, a derivative or fragment thereof according to claims 1-2, in combination with an anti-TNF-alpha antibody and/or TGF-beta.

4. The use according to claim 1, wherein said activated uPAR is a uPAR fragment containing the YRSRY motif.

5. The use according to claim 4, wherein the uPAR fragments are selected from the group consisting of peptides 84-279, 85-279, 86-279, 87-279, 88-279 and 89-279, referred to uPAR amino acid sequence.

6. The use according to claim 4, wherein the u-PAR fragments are selected from the group consisting of peptides AVTYSRSRYLEC, VTYSRSRYLEC, TYSRSRYLEC, YSRSRYLEC.

7. The use according to any previous claims, for the treatment of acute or latent HIV-infections in HIV-infected individuals or in AIDS patients.

8. A method for the screening of compounds useful for the treatment of HIV-infection, which comprises:
a) selecting a compound able to modulate the pro-uPA/uPAR interaction,
b) contacting said compound with a culture of U1 promonocytic cells in which HIV-production has been suitably activated,
c) determining any variations of virus replication

9. A method according to claim 8, wherein the HIV-production by U1 cells is activated by their stimulation with TNF-alpha, IL-6 or IL-1beta.

10. A method for the determination of activated uPAR from tissue, tissue-extracts, biological fluids, of HIV infected individuals, which comprises:
a) preparing a sample of tissue, tissue-extracts, biological fluids;
b) contacting that sample with an antibody against the AVTYSRSRYLEC peptide;
c) determining the specific binding of the antibody.

11. A method according to claim 10, which is in form of ELISA.

12. A method according to claims 10-11, for the ex-vivo diagnosis or prognosis of HIV infection.
